# EUROPEAN PATENT SPECIFICATION

(11) **EP 4 541 299 B1**
(45) Date of publication and mention of the grant of the patent: **07.10.2026**
(21) Application number: 24220128.3
(22) Date of filing: 17.12.2015
(51) Int. Cl.: A61B 18/14, A61B 17/29, A61B 18/00

(54) **BIPOLAR ELECTROSURGICAL SEALER AND DIVIDER**
BIPOLARES ELEKTROCHIRURGISCHES VERSIEGELUNGS- UND TRENNGERÄT
OBTURATEUR ÉLECTROCHIRURGICAL BIPOLAIRE ET DIVISEUR

(30) Priority: 23.12.2014 US 201462096255 P
(43) Date of publication of application: 23.04.2025
(60) Divisional application: 26201580.3
(62) Document number(s) of the earlier application(s) in accordance with Art. 76 EPC: 19198318.8 / 3 603 557; 15820790.2 / 3 236 870
(73) Proprietor: Applied Medical Resources Corporation, Rancho Santa Margarita, CA 92688 (US)
(72) Inventor: LE, Richard, Rancho Santa Margarita, 92688 (US)
(74) Representative: Dolleymores

(56) References cited:
- WO-A1-2009/003575
- US-A1- 2008 015 566
- US-A1- 2014 214 019

## Description

### Background

The present application relates to an electrosurgical instrument and more particularly relates to a bipolar electrosurgical sealer and divider instrument.

Electrosurgical instruments or tools have become available that use electrical energy to perform certain surgical tasks. Typically, electrosurgical tools are hand tools such as graspers, scissors, tweezers, blades, needles, and other hand tools that include one or more electrodes that are configured to be supplied with electrical energy from an electrosurgical generator including a power supply. The electrical energy can be used to coagulate, fuse, or cut tissue to which it is applied.

Electrosurgical tools typically fall within two classifications: monopolar and bipolar. In monopolar tools, electrical energy of a certain polarity is supplied to one or more electrodes on the tool. A separate return electrode is electrically coupled to a patient. Monopolar electrosurgical tools can be useful in certain procedures, but can include a risk of certain types of patient injuries such as electrical burns often at least partially attributable to functioning of the return electrode. In bipolar electrosurgical tools, one or more electrodes is electrically coupled to a source of electrical energy of a first polarity and one or more other electrodes is electrically coupled to a source of electrical energy of a second polarity opposite the first polarity. Thus, bipolar electrosurgical tools, which operate without separate return electrodes, can deliver electrical signals to a focused tissue area with reduced risks.

Even with the relatively focused surgical effects of bipolar electrosurgical tools, however, surgical outcomes are often highly dependent on surgeon skill. For example, thermal tissue damage and necrosis can occur in instances where electrical energy is delivered for a relatively long duration or where a relatively high-powered electrical signal is delivered even for a short duration. The rate at which a tissue will achieve the desired coagulation or cutting effect upon the application of electrical energy varies based on the tissue type and can also vary based on pressure applied to the tissue by an electrosurgical tool. However, even for a highly experienced surgeon, it can be difficult for a surgeon to assess how quickly a mass of combined tissue types grasped in an electrosurgical instrument will be fused a desirable amount.

Attempts have been made to reduce the risk of tissue damage during electrosurgical procedures. For example, previous electrosurgical systems have included generators that monitor an ohmic resistance or tissue temperature during the electrosurgical procedure, and terminated electrical energy once a predetermined point was reached. However, these systems have had shortcomings in that they have not provided consistent results at determining tissue coagulation, fusion, or cutting endpoints for varied tissue types or combined tissue masses. These systems can also fail to provide consistent electrosurgical results among use of different tools having different tool and electrode geometries. Typically, even where the change is a relatively minor upgrade to tool geometry during a product's lifespan, the electrosurgical generator must be recalibrated for each tool type to be used, a costly, time consuming procedure which can undesirably remove an electrosurgical generator from service. Examples of known electrosurgical instruments are disclosed in patent applications bearing the publication numbers US 2014/214019 A1, WO 2009/003575 A1 and US 2008/0015566 A1.

### Summary

In accordance with the present invention there is provided an electrosurgical instrument as recited in Claim 1.

Features of the invention will become more apparent with a discussion of embodiments in reference to the associated drawings.

### Brief Description of Drawings

The present inventions may be understood by reference to the following description, taken in connection with the accompanying drawings in which the reference numerals designate like parts throughout the figures thereof.
FIG. 1 is a perspective view of an electrosurgical generator for use with a surgical instrument in accordance with the present invention.
FIG. 2 is a perspective view of an electrosurgical fusion/sealer and dissector instrument in accordance the present invention.
FIG. 3 is a side view of an electrosurgical fusion/sealer and dissector instrument in accordance with the present invention.
FIG. 4 is an exploded view of an electrosurgical fusion/sealer and dissector instrument in accordance with the present invention.
FIG. 5 is a perspective view of an electrosurgical fusion/sealer and dissector instrument in accordance with the present invention.
FIG. 6 is a side view of an electrosurgical fusion/sealer and dissector instrument in accordance with the present invention.
FIG. 7 is a side view of an electrosurgical fusion/sealer and dissector instrument in accordance with the present invention.
FIG. 8 is a side view of an electrosurgical fusion/sealer and dissector instrument in accordance with the present invention.
FIG. 9 is a side view of an electrosurgical fusion/sealer and dissector instrument in accordance with the present invention.
FIG. 10 is a side view of an electrosurgical fusion/sealer and dissector instrument in accordance with the present invention.
FIG. 11 is a side view of an electrosurgical fusion/sealer and dissector instrument in accordance with the present invention.
FIG. 12 is a side view of an electrosurgical fusion/sealer and dissector instrument in accordance with the present invention.
FIG. 13 is a side view of an electrosurgical fusion/sealer and dissector instrument in accordance with the present invention.
FIG. 14A is a side view of a distal end of an electrosurgical fusion/sealer and dissector instrument in accordance with the present invention.
FIG. 14B is a side view of an electrosurgical fusion/sealer and dissector instrument in accordance with the present invention.
FIG. 15 is a cross sectional view of an electrosurgical fusion/sealer and dissector instrument in accordance with the present invention.
FIG. 16 is a side view of an electrosurgical instrument in accordance with the present invention.

### Detailed Description

Generally, an electrosurgical system is provided that includes an electrosurgical generator and an electrosurgical tool that are configured to optimally fuse tissue. The electrosurgical tool in accordance with invention is provided to be used in open surgery with the ability to move, grasp and compress tissue and to deliver RF energy to fuse tissue. The electrosurgical tool is a bipolar electrosurgical sealer and divider tool that in various embodiments may not be insertable through a 5mm-12mm laparoscopic cannula but is typically used in open surgeries or through devices able to accommodate the non-cylindrical and larger than 12mm distal end of the electrosurgical tool. RF energy is supplied by the electrosurgical generator configured to provide the appropriate RF energy to fuse tissue. The generator may determine the appropriate RF energy and the appropriate manner to deliver the RF energy for the particular connected electrosurgical tool, the particular tissue in contact with the tool and/or a particular surgical procedure. Information or data to assist in the determination of the appropriate RF energy and manner to deliver the RF energy may be supplied or obtained externally from the generator. The external source may comprise one or more memory modules that may be included with the electrosurgical tool or via connections therebetween (wired or wireless) or via a separate tool, accessory, adapter and/or connections therebetween and/or via a separate port or connection to the generator. The generator retrieves and/or receives the data and utilizes the data to command or operate the generator to determine and supply the appropriate RF energy in the appropriate manner.

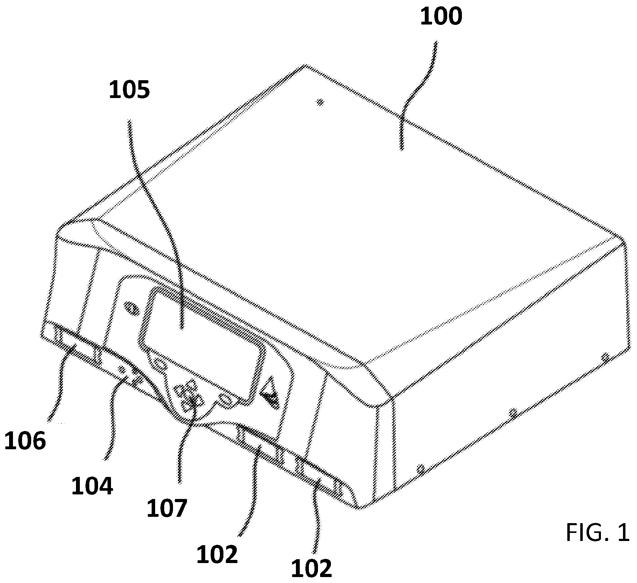

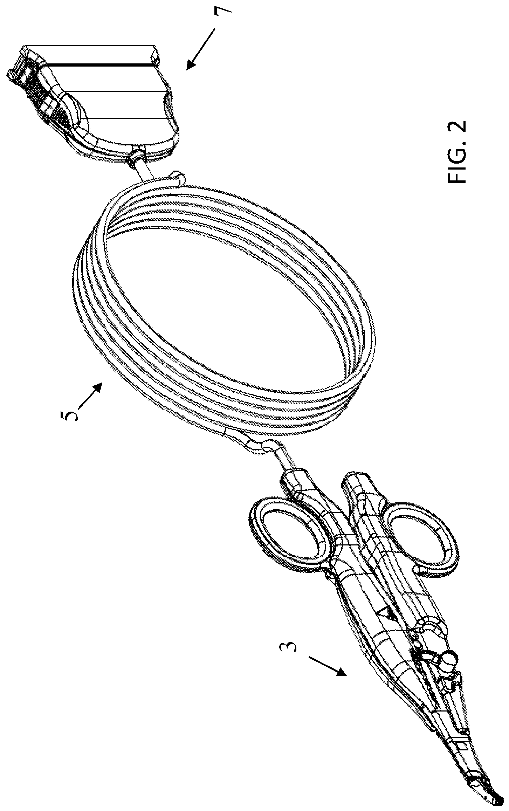

Referring to FIGS. 1-2, an exemplary electrosurgical system is illustrated including an electrosurgical generator 100 and a removably connectable electrosurgical tool or instrument 3 in accordance with the present invention. The electrosurgical instrument 3 can be electrically coupled to the generator via a cabled connection 5 and tool plug or connector 7 to a tool port 102 on the generator. The electrosurgical instrument 3 may include audio, tactile and/or visual indicators to apprise a user of a particular predetermined status of the tool such as a start and/or end of a fusion operation. The electrosurgical instrument can be reusable after sterilization and/or connectable to another electrosurgical generator for another surgical procedure. A manual controller such as a hand or foot switch can be connectable to the generator and/or tool to allow predetermined selective control of the tool such as to commence a fusion operation.

The electrosurgical generator 100 is configured to generate radiofrequency (RF) electrosurgical energy and to receive data or information from the electrosurgical instrument electrically coupled to the generator. The generator 100 outputs RF energy (e.g., 375VA, 150V, 5A at 350kHz) and is configured to calculate a phase angle or difference between RF output voltage and RF output current during activation or supply of RF energy. The generator regulates voltage, current and/or power and monitors RF energy output (e.g., voltage, current, power and/or phase). The generator 100 stops RF energy output under predefine conditions such as when a device switch is de-asserted (e.g., fuse button released), a time value is met, and/or active phase angle and/or change of phase is greater than or equal to a phase and/or change of phase stop value indicating end of an operation such as fusion of tissue between the jaws.

The electrosurgical generator 100 comprises two advanced bipolar tool ports 102, a standard bipolar tool port 106, and an electrical power port 104, but electrosurgical units can comprise different numbers of ports. For example, an electrosurgical generator can comprise more or fewer than two advanced bipolar tool ports, more or fewer than the standard bipolar tool port, and more or fewer than the power port. In one embodiment, the electrosurgical generator comprises only two advanced bipolar tool ports.

Each advanced bipolar tool port 102 is configured to be coupled to electrosurgical instrument having an attached or integrated memory module. The standard bipolar tool port 106 is configured to receive a non-specialized bipolar electrosurgical tool that differs from the advanced bipolar electrosurgical instrument connectable to the advanced bipolar tool port 102. In one embodiment, the non-specialized bipolar electrosurgical tool does not include or is not connectable to a memory module that may have operational or parameter data for the operation of the tool. The electrical power port 104 is configured to receive or be connected to a direct current (DC) accessory device that differs from the non-specialized bipolar electrosurgical tool and the advanced electrosurgical instrument. The electrical power port 104 is configured to supply direct current voltage. For example, the power port 104 can provide approximately 12 Volts DC. The power port 104 can be configured to power a surgical accessory, such as a respirator, pump, light, or another surgical accessory. Thus, in addition to replacing electrosurgical generator for standard or non-specialized bipolar tools, the electrosurgical generator can also replace a surgical accessory power supply. Replacing presently-existing generators and power supplies with the electrosurgical generator can reduce the amount of storage space required on storage racks carts or shelves and in the number of mains power cords required in a surgical workspace.

Connection of a non-specialized bipolar instrument into the standard bipolar port will not cause the generator to actively check the tool. However, the generator recognizes a connection so that the information of the non-specialized bipolar tool can be displayed. The generator recognizes device connection status for each of the advanced tool ports 102 and authenticates connected devices before accepting RF energy activation requests (e.g., activation of a tool switch such as a fuse button). The generator may read authenticated data from the connected device and reads electrical control values (such as but not limited to voltage level settings, current level settings, power level settings, active phase angle level settings, RF energy output activation timing limits, tool short limits, tool open limits, tool model/identification, RF energy output line configurations, switch state command configurations and/or combinations thereof) from the authenticated and connected device.

The electrosurgical generator 100 can comprise a display 105. The display can be configured to indicate the status of the electrosurgical system including, among other information, the status of the one or more electrosurgical tools and/or accessories, connectors or connections thereto. In some embodiments, the display can comprise a multi-line display capable of presenting text and graphical information such as for example an LCD panel display, which, in some embodiments can be illuminated via backlight or sidelight. In some embodiments, the display can comprise a multi-color display that can be configured to display information about a particular tool electrically coupled to the electrosurgical generator and a color that corresponds to a particular surgical procedure (such as, for example cutting operations displayed in yellow text and graphics, fusion or welding operations displayed in purple, and coagulation displayed in blue, bloodless dissection operations can be displayed in yellow and blue). In some embodiments, the display can be configured to simultaneously indicate status data for a plurality of tools electrically coupled to the electrosurgical generator and/or be portioned to display status information for each tool connected to a corresponding tool port. A visual indicator such as a status bar graph can be used to illustrate a proportion of total available electrical energy to be applied to the bipolar electrosurgical tool when actuated. In various embodiments, an electrosurgical tool operable to cut, coagulate, or fuse tissue could have three color-coded displays or bar graphs. In some embodiments, a user can toggle the display between presenting status of multiple electrically connected tools and status of a single electrically connected tool. In accordance with various embodiments, once a tool and/or accessory is connected and/or detected a window opens in the user interface display showing the type of tool connected and status.

The electrosurgical generator can comprise a user interface such as, for example a plurality of buttons 107. The buttons can allow user interaction with the electrosurgical generator such as, for example, requesting an increase or decrease in the RF energy supplied to one or more tools coupled to the electrosurgical generator. In other embodiments, the display 105 can be a touch screen display thus integrating data display and user interface functionalities. Through the user interface, the surgeon can set a voltage setting by the selection of one to three levels. For example, at level 1, voltage is set to 110V; at level 2, voltage is set to 100V; and at level 3, voltage is set to 90V. Current is set to 5Amps and power is set to 300 VA for all three levels. In other embodiments, the voltage is preset or defaults to a specific level such as level 2. In other embodiments, like the current and power settings, the voltage setting is not user adjustable to simplify operation of the generator and as such a predetermined default voltage setting is utilized, e.g., voltage is set to 100V.

The electrosurgical instrument can further comprise one or more memory modules. In some embodiments, the memory comprises operational data concerning the tool and/or other tools. For example, the operational data may include information regarding electrode configuration/reconfiguration, the tool uses, operational time, voltage, power, phase and/or current settings, and/or particular operational states, conditions, scripts, processes or procedures. The generator initiate reads and/or writes to the memory module.

Each advanced bipolar electrosurgical tool comes with a memory module and/or an integrated circuit that provides tool authentication, configuration, expiration, and logging. Connection of such tools into the receptacles or ports initiates a tool verification and identification process. Tool authentication is provided via a challenge-response scheme and/or a stored secret key also shared by the generator. Other parameters have hash keys for integrity checks. Usages are logged to the generator and/or to the tool integrated circuit and/or memory. Errors can result in unlogged usage. The log record is set in binary and interpreted with offline tools or via the generator.

The generator uses time measurement components to monitor a tool's expiration. Such components utilize polling oscillators or timers or real-time calendar clocks that are configured at boot time. Timer interrupts are handled by the generator and can be used by scripts for timeout events. Logging also utilizes timers or counters to timestamp logged events.

The generator provides the capability to read the phase difference between the voltage and current of the RF energy sent to the connected electrosurgical tool while RF energy is active. While tissue is being fused, phase readings are used to detect different states during the fusion process.

The generator logs usage details in an internal log that is down loadable. The generator has memory for storage of code and machine performance. The generator also has reprogrammable memory that contains instructions for specific tool performance. The memory for example retains a serial number and tool use parameters. The generator may also store information on the type of tools connected. Such information includes but is not limited to a tool identifier, e.g., a serial number of a connected tool, along with a time stamp, number of uses or duration of use of the connected tool, power setting of each and changes made to the default setting. The memory in one embodiment holds data for about two months or about 10,000 tool uses and is configured to overwrite itself as needed.

The generator does not have to monitor or control current, power or impedance. The generator regulates voltage and can adjust voltage. Electrosurgical power delivered is a function of applied voltage, current and tissue impedance. The generator through the regulation of voltage can affect the electrosurgical power being delivered. However, by increasing or decreasing voltage, delivered electrosurgical power does not necessarily increase or decrease. Power reactions are caused by the power interacting with the tissue or the state of the tissue without any control by a generator other than by the generator supplying power.

The generator once it starts to deliver electrosurgical energy does so continuously until a fault occurs or a specific phase parameter is reached. In one example, the jaws of the electrosurgical tool can be opened and thus compression relieved at any time before, during and after the application of electrosurgical energy. The generator also does not have to pause or wait a particular duration or a predetermined time delay to commence termination of the electrosurgical energy.

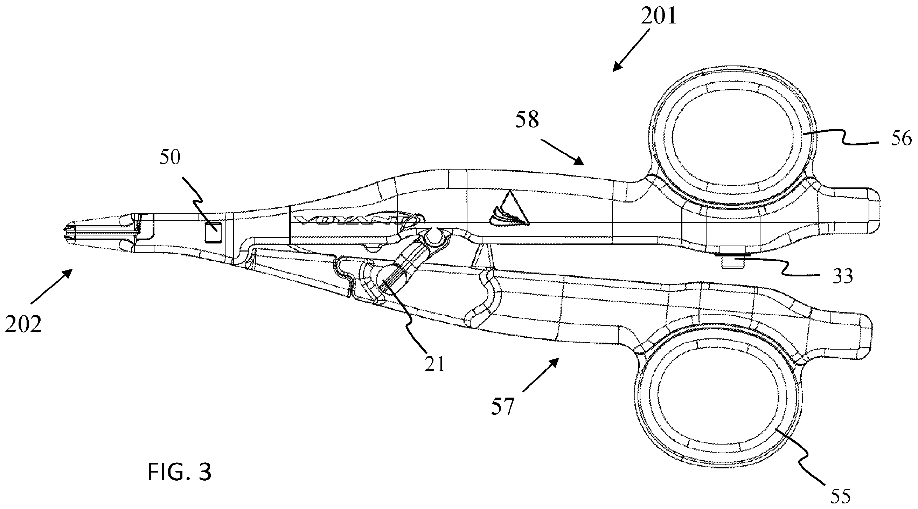

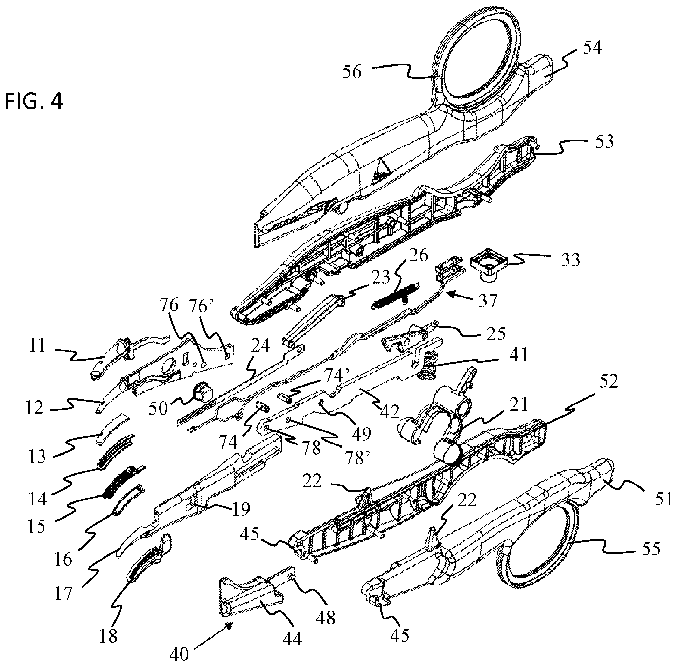

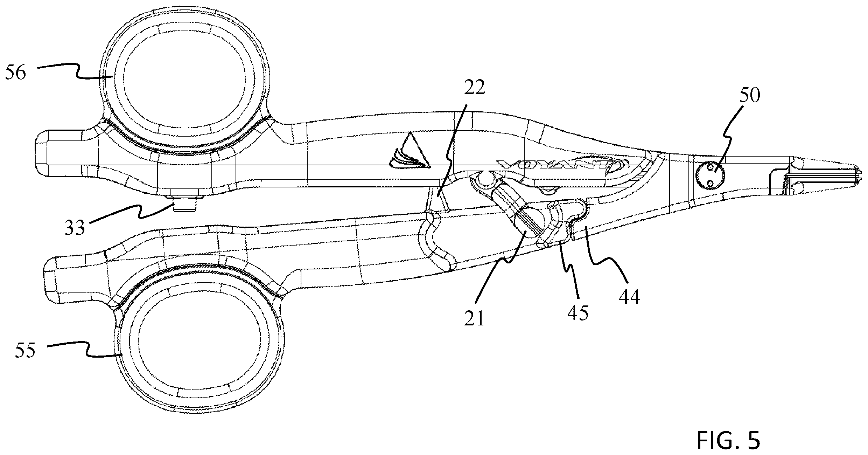

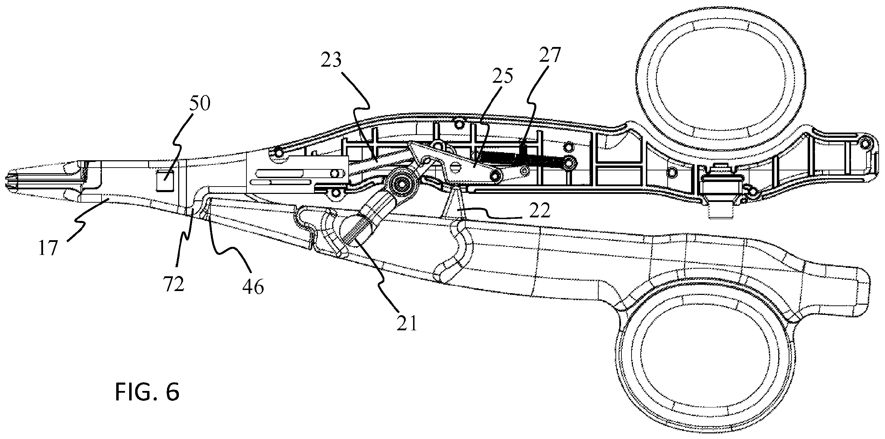

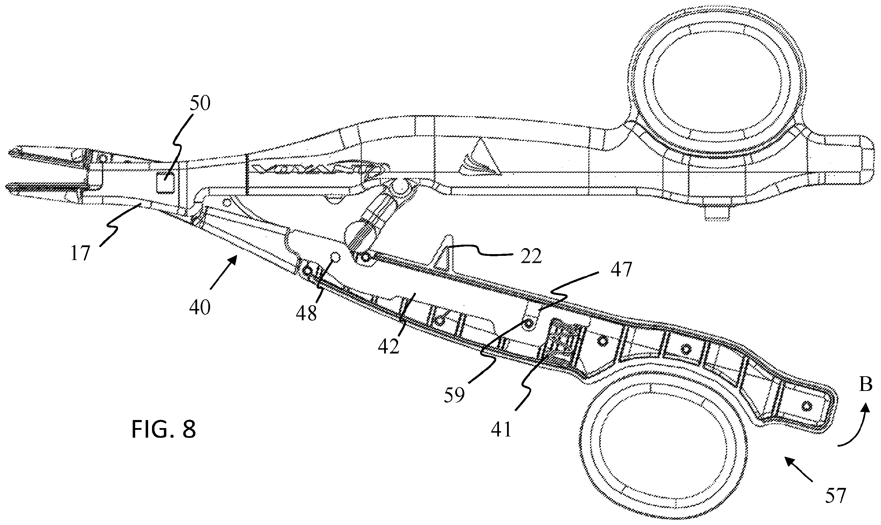

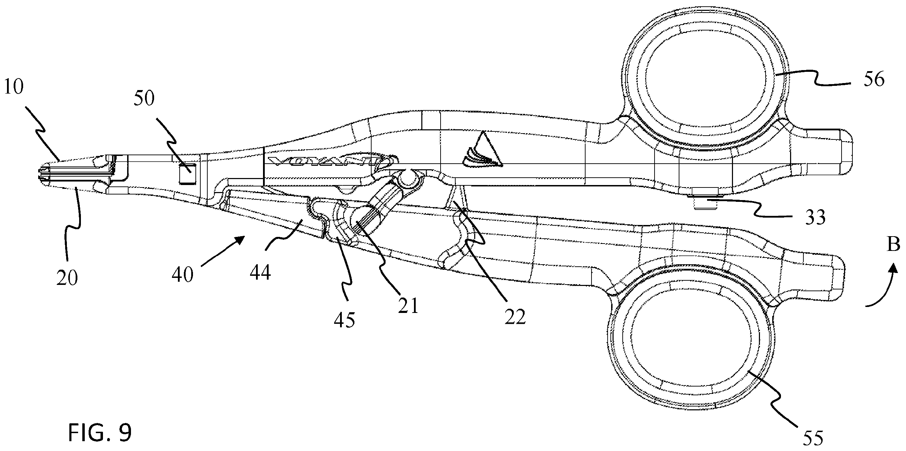

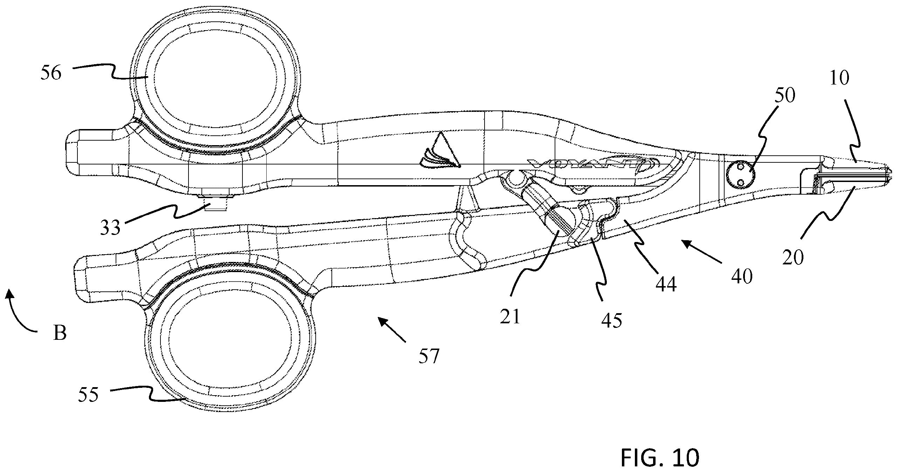

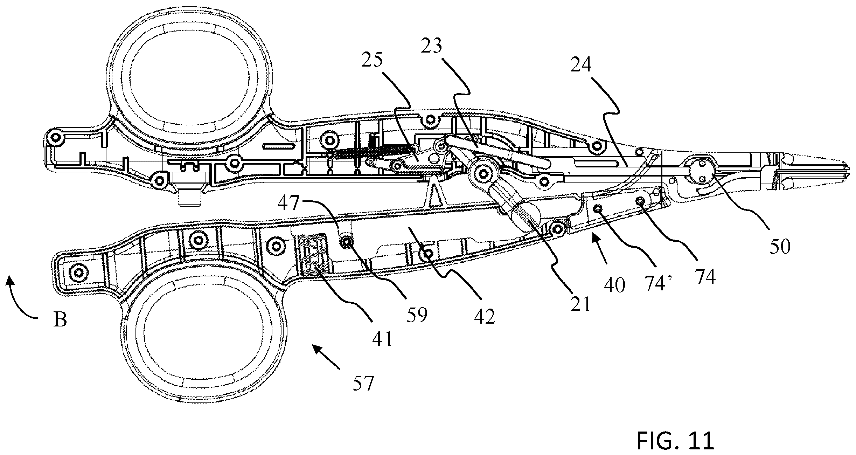

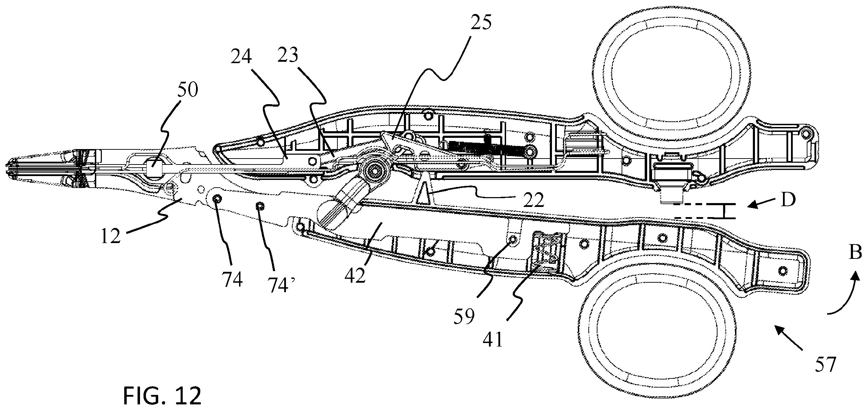

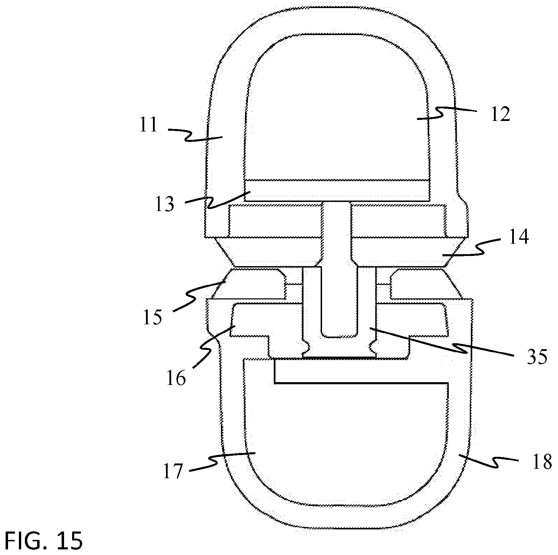

With reference also to FIGS. 3-15, an electrosurgical instrument is disclosed, which is in accordance with the present invention. In the illustrated embodiment, the instrument includes an actuator 201 coupled to or from which jaws 202 extend. In one embodiment, the actuator 201 includes two finger loops 55, 56 each extending from a different handle 57, 58 with both handles movable relative to each other. Both handles are pivotally coupled together through a central or main pivot 50. In operation, the handles are manipulated by a user, e.g., a surgeon, to move the jaws, selectively opening and closing the jaws.

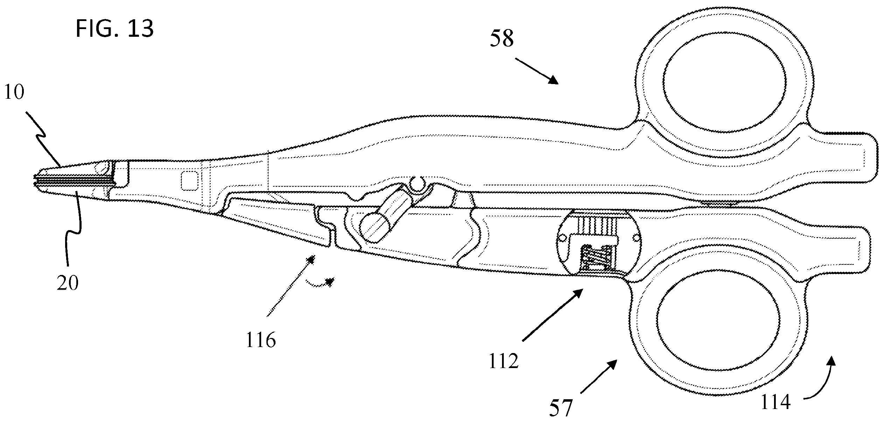

A fuse/seal switch or button 33 is activated upon complete closure of the handles as shown in at least FIG. 13 and indicated by arrow D. The fuse button 33 can, but is not operationally pushed by a surgeon. In operation, the jaws are arranged to be fully closed grasping and compressing tissue between the jaws while the seal button 33 remains inactivated. The handles are closed further at which time a force regulation and over-compression or spring arm mechanism, as will be described in greater detail below, ensures a predetermined compression force or range of compression forces is applied or at most applied on the tissue between the jaws. Once the handles are fully closed, first or lower handle 57 or an outer surface of portion of handle 57 engages the fuse button 33. As such, as the handle 57 moves proximate to the other opposing handle, second or upper handle 58, while the jaws still remain fully closed, the seal button 33 between the handles is depressed. Upper handle 58, in one embodiment, comprises of upper handle housing 53, 54 that forms a cavity in which portions of the fuse button are captured within. In one embodiment, the fuse button has a first state indicating contact or providing tactile indication (such as increased friction, force or resistance), visual and/or audio indications to a surgeon that the button has been contacted and a second state with similar indications that indicate the button has been fully depressed and thus fusion of tissue between the jaws have been activated or started and thus RF energy is being applied to the tissue. Once the fuse button is activated, associated circuitry or contacts are connected to connect appropriate electrodes of the jaws with associated connections of the generator to supply RF energy to fuse tissue grasped between the jaws.

The instrument comprises a translatable mechanical cutting blade that is coupled to a blade actuator, such as, blade trigger or lever 21 of the actuator 201. The mechanical cutting blade is actuated by the blade lever 21 to divide tissue between the jaws. A blade lock hook 25 within one of the handles prevents movement of the blade lever 21 but upon closure of the handles/jaws the blade lock hook releases the blade lever 21. The blade hook when engaged by a blade unlock projection 22 in the other handle releases the blade lever allowing the blade lever to be moved and thus to actuate the blade through the jaws and the tissue grasped between the jaws.

The main or central pivot 50 about which the handles and jaws pivot has an opening through which a blade slider 24 is extendable and retractable there through and thus the pivot 50 does not hinder or restrict the blade slider regardless of the position of the pivot. In one embodiment, the main pivot 50 has a circular end inserted and connected to a circular opening in an upper or second jaw support 12 and a square, rectangular or non-circular end inserted and connected to a square, rectangular or non-circular opening 19 in a lower or first jaw support 17. The circular connection between the central pivot 50 and the upper jaw support 12 allows the upper jaw support to pivot about the central pivot. Conversely, the square or non-arcuate connection between the central pivot 50 and the lower jaw support 17 resists rotation or pivoting of the lower jaw support about the central pivot 50. Accordingly, the upper or second jaw moves from a proximate position to a spaced position and vice versa relative to the non-movable or relatively stationary lower jaw as the lower handle is moved towards the upper handle rotating about the central pivot 50.

The blade slider 24 is connected to a blade lever arm 23. The blade lever arm 23 is connected to the blade trigger 21. In one embodiment, a projection such as a pin extends from a distal portion of the blade lever arm 23 into an opening in a proximal end of the blade slider 24 connecting the components together. In one embodiment, a projection extends from a proximal portion of the blade lever arm 23 into an opening in one end of the blade trigger 21 connecting the components together. The other end of the blade trigger 21 is exposed and accessible by the user with the blade trigger being pivotable about a trigger pivot at or near the mid-point of the blade trigger. As such, as the blade trigger is pulled or rotated by the user proximally or counterclockwise, the end of the blade trigger 21 connected to the blade lever arm 23 moves distally which simultaneously moves the blade lever arm distally. The blade lever arm 23 connected to the blade slider 24 slides or moves the blade slider distally. Integrated with or attached to a distal end of the blade slider is a cutting blade, knife or cutting edge or surface. As such, as the blade slider 24 translates longitudinally through a channel in the jaws, tissue grasped between the jaws is cut. In one embodiment, the cutting edge or surface is angled to facilitate cutting of the tissue between the jaws. In various embodiments, the cutting blade is a curved blade, a hook, a knife, or other cutting elements that is sized and configured to cut tissue between the jaws.

A spring 26 connected to the blade trigger 21 biases the blade trigger back proximally and thus when the blade trigger is released by the surgeon, the blade trigger rotates or pivots back to its initial position. In one embodiment, a spring 27 is connected to the blade lever arm that biases the blade lever arm and the blade slider connected thereto back to the initial or retracted position. Hence, once the blade trigger is released, the blade slider translates longitudinally back proximally through the channel in the jaws to its initial position. Upon return, the blade trigger engages the blade lock hook 25 moving or lifting the hook as the trigger continues to proceed back to its initial or starting position. Once back to the initial position, the blade hook is free to move back and engage the trigger holding it in place if not obstructed by the unlock projection 22.

In one embodiment, the lower jaw support 17 includes a guide channel that is dimensioned to receive and support the blade slider 24 to ensure alignment and support longitudinal translation of the slider distally and/or proximally. In one embodiment the lower jaw support includes a pin channel at a proximal end of the lower jaw support away from the lower jaw or distal end of the instrument. The proximal pin channel is dimensioned to receive and support the pin or connection between the blade slider 24 and the blade lever arm 23 as the pin, blade slider and the blade lever arm translates distally and proximally and to align and support longitudinal translation of the slider and pin distally and/or proximally.

The jaws are opened and closed by corresponding movements of the handles or handle connected to a respective jaw. The jaws and handles are movable through at least three states, conditions or positions. In a first or initial (or open) position, the jaws are opened with the upper and lower jaws spaced from each other and the handles are open with the upper and lower handles being spaced from each other. In a second (or clamped) position, the jaws are closed with the upper and lower jaws proximate to each other and the handles are closed with the upper and lower handles being proximate to each other. In a third (or fuse) position, the jaws remain closed as in the second position but the handles are fully closed with the upper and lower handles having portions that contact or interact with each other (e.g., a fuse button contacted or activated). The handles and jaws are movable between each of the three positions. The jaws, when closed upon tissue, apply pressure or compression to the tissue between the jaws. This compression corresponds to the closure force being applied by the surgeon. In accordance with various embodiments, the instrument includes a force regulation and over-compression or spring arm mechanism that applies the proper compression while not over squeezing or applying too much compression due to differences in operational use and/or size variations of the tissue. In various embodiments, the force regulation and over compression mechanism is or is only activated or operates when the jaws and/or handles are moved from the second position to the third position.

In accordance with various embodiments, the instrument applies a predetermined pressure or range of pressures by allowing one of the device handles to control the clamping pressure through an internal support spring 41. As the two handle close together and bottoms out, one handle is able to dislocate or decouple rotationally from the jaws and a rocking motion is introduced. The rocking motion in one embodiment is the result of the handle 57, jaw support joint 40 and mating contours 44, 45 interacting with each other. As the handle and in particular the handle housing dislocates or decouples from the jaws and in particular the support joint, the internal spring 41 compresses which subjects a jaw support arm 42 to a controller moment when the jaws are fully closed. With the support spring controlling the clamping pressure instead of a fixed rigid lever, for example, the instrument is able to maintain and ensure a particular clamping pressure range. Additionally, any further pressure or movement by the handles as applied by the surgeon is removed or decoupled from the jaws. As such, the handle is dislocated or decoupled rotationally from the jaws which allows the handles to move to a fully closed or fuse position without creating additional surface pressure load or compression on the tissue/vessel.

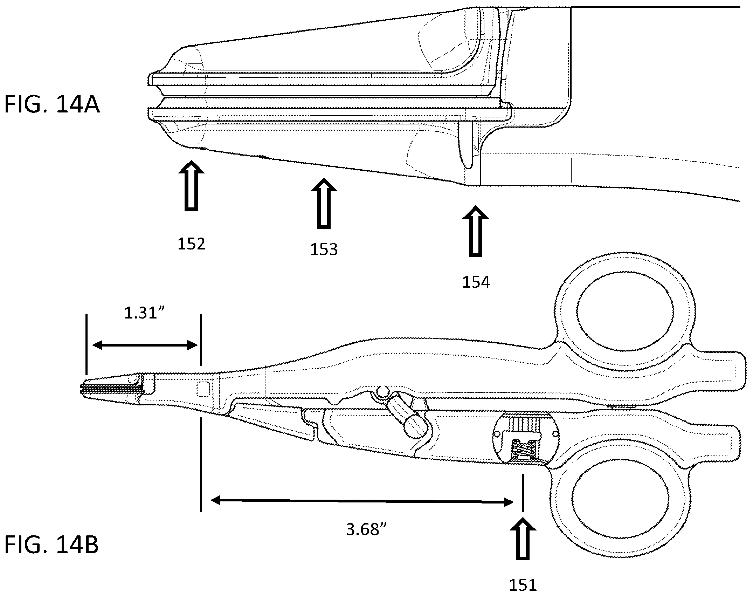

The support spring 41 supplies the set pressure or force that allows the instrument to target or set a predetermined optimal sealing pressure as shown in FIGS. 14A-B. In particular, a spring applying 8.09 N (2 pounds of force) on the support arm when the handles bottom out (arrow 151) applies a range of pressure of 26.69 N (6 lbs.) (arrow 152) to 40.71 N (10.5 lbs.) (arrow 154) at the jaws with about 33.36 N (7.5 lbs.) (arrow 153) at the jaws' midpoint. Likewise, a spring applying 14.46 N (3.25 pounds of force) on the support arm when the handles bottom out (arrow 151) applies a range of pressure of 41.15 N (9.25 lbs.) (arrow 152) to 71.17 N (16 lbs.) (arrow 154) at the jaws with about 53.38 N (12 lbs.) (arrow 153) at the jaws' midpoint. The distance between the spring and the pivot 50 (e.g., 9.35 cm (3.68 inches)) and the distance between the pivot 50 and the end of the jaws or sealing surface (e.g., 3.33 cm (1.31 inches)) remain constant. As such, the controlled forces applied by the instrument vary along the length of the jaws from about 24.47 N (5.5 pounds of force) near the distal tip or portion in various embodiments to about 71.17 N (16 pounds of force) near the proximal portion of the jaws in various embodiments. In accordance with various embodiments, a force ratio from the jaw arm to the jaw tip is a ratio of 2.81:1. The spring 41 is also not exposed or accessible by the surgeon and thus prevents potential interference with a surgeon's operation, catching on a surgeon's gloves, misalignment, interference and/or harm to the mechanism, and increases ease of assembly and manufacture.

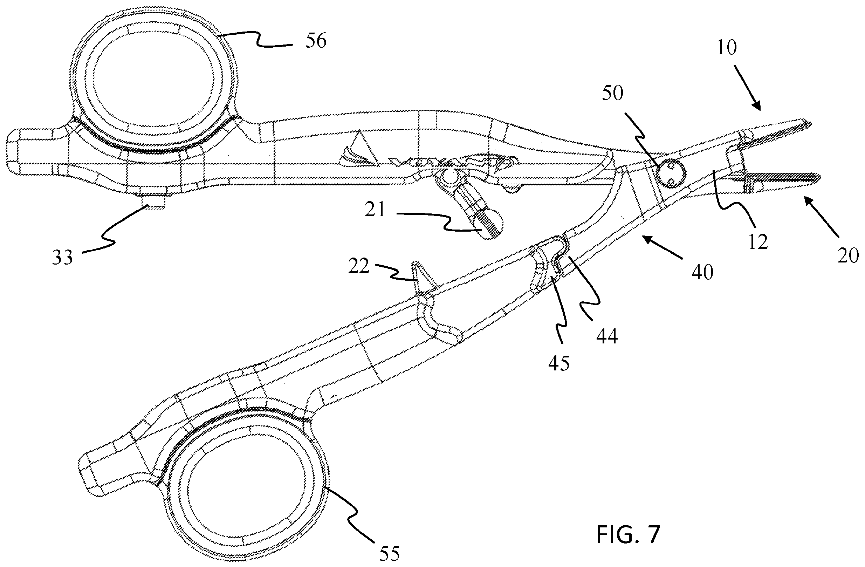

In accordance with various embodiments, a surgeon can open and close the jaws (upper and lower jaws 10, 20) of the electrosurgical instrument to move and grasp tissue there between. Once the surgeon grasps tissue to be sealed and/or cut, the surgeon will continue to rotate and close the handles to unlock the blade lever and engage the fuse button 33 (FIG. 7). Additionally, further movement of the lower or bottom handle 57 in the rotational direction indicated by the arrow B causes the housing of the bottom handle 57 to decouple or break away from the jaw support joint 40. Corresponding mating curved surfaces or contours 44, 45 provides external support to assist in the decoupling of the housing of the bottom handle from the jaw support joint 40 and facilitate or introduce a rocking motion between the jaw support joint 40 and the lower handle 57. In various embodiments, the jaw support joint has a curved cutout 46 that mates with a curved surface 72 of the lower jaw support to further facilitate the rocking motion and/or the pivoting of the handles relative to each other.

The pivot or jaw support arm 42 is connected to the lower or bottom handle 57. The bottom handle 57 defines an enclosed cavity and in one embodiment the bottom handle 57 comprises lower left and right handle housing 51, 52 that define the enclosed cavity therebetween. Near the proximal end of the jaw support arm 42 is the support spring or biasing member 41. A slot 47 is also formed near the proximal end of the jaw support arm 42 and the support spring 41 is connected to the proximal end of the jaw support arm 42 near the slot. The spring and slot are enclosed in the cavity of the bottom handle 57. The spring and slot extends along parallel axes and are perpendicular or traverse to the longitudinal axis of the instrument (e.g., an axis through which the blade extends and retracts) and/or the jaw support arm 42. A pin 59 extending from within the bottom handle 57 extends through the slot or channel 47 in the jaw support arm. In one embodiment, from an initial or open position to a close position in which the jaws are open and closed respectively, the pin is near the bottom or closed end of the slot and the spring 41 remains uncompressed. As the handles are fully closed, bottomed out and moved to a third fuse position, the pin 59 of the handle moves from the bottom end of the slot towards the upper end of the slot and the spring 41 compresses. At the distal end of the jaw support arm 42, the jaw support arm is connected to the upper jaw support 12 via projections, such as pins or posts 74.

In accordance with various embodiments, the jaw support joint 40 includes a cavity in which a distal portion of the jaw support arm 42 is received and connected to the jaw support joint 40. In one embodiment, one or more pins 74 extend through apertures 78 at the distal end of the jaw support arm and into receptacles or openings in the jaw support joint to secure the jaw support arm 42 to the jaw support joint 40. In one embodiment, the one or more pins 74 also extend through apertures 76 at the proximal end of the upper jaw support 12 securing the upper jaw support 12 to the jaw support joint 40 and the support arm 42.

The jaw support joint 40 in one embodiment at the proximal end or portion is a projection or post 48 that extends into an opening 49 in the jaw support arm 42 thereby further connecting the jaw support joint to the jaw support arm. The opening in the jaw support arm is between the proximal and distal end of the support arm. A portion of the proximal end of the jaw support joint also extends into the handle housings 51, 52 thereby capturing and securing the jaw support joint to the handle 57. The jaw support arm 42 connected to the jaw support joint 40 remains fixed and thus further pivoting or rotational movement of the jaw support arm 42 is prevented even though the bottom handle may continue to rotate under pressure of the user further closing the handles together. As the bottom handle 57 continues to rotate after the jaws are closed, the support spring 41 is compressed (arrow 112) by the interaction of the jaw support arm 42 being fixed and the bottom handle 57 rotating or moving (arrow 114).

When the handles bottom out, the support spring 41 inside the lower handle applies a specific force on the jaw support arm 42 which translates to a specific or controlled clamping force at the jaws. The lower handle housing breaking away from the jaw support arm 42 disassociates or dislocates (arrow 116) any additional compression applied by the user through the squeezing of the handles together. At the dislocation or decoupling operational point, in accordance with various embodiments, the internal support spring 41 is engaged or activated. As such, prior to that dislocation operational point the spring is not engaged and thus the surgeon can open and close the jaws and grasp or dissect different tissue to seal, reposition or move out of the way without having to overcome a spring force with each movement. This reduces or prevents hand fatigue. Also, potential trauma to tissue not intended to be fused is avoided through the unintended application of force by the instrument for merely grasping or moving tissue. In one embodiment, the support spring 41 provides or supplies the only clamping force at the jaws with force supplied by the surgeon's squeezing of the handles being dissociated or decoupled from the jaws.

The jaws include upper and lower jaws with both jaws having an electrode or conductive pad. The conductive pad 14 of the upper jaw and the conductive pad 15 of the lower jaw are electrically coupled to the electrosurgical generator via wires and connectors 37 to supply RF energy to tissue grasped between the conductive pads. The conductive pads are arranged to have opposing polarity. The upper jaw includes an upper jaw support 12 with an assembly spacer 13 positioned between the upper jaw support 12 and the conductive pad 14. The upper jaw also includes an overmold 11 or is overmolded. The lower jaw includes a lower jaw support 17 with an assembly spacer 16 positioned between the jaw support 17 and the conductive pad 15.

In various embodiments, a blade channel extends longitudinally along the length of the upper jaw, the lower jaw or both through which the blade operationally traverses. The lower jaw also includes an overmold 18 or is overmolded. Surrounding a portion of the blade channel is one or more conductive posts 35. The conductive posts assist in strengthening the blade channel and support the tissue to be cut. The conductive posts also assist in ensuring the tissue being cut adjacent or proximate to the blade channel is fused as the conductive posts also participate in the transmission of RF energy to the tissue grasped between the jaws.

In one embodiment, wires and associated connections 37 extend from the fuse switch or button 33 through the upper housing, upper and lower jaw supports to the respective upper and lower jaws and the respective connections to the upper and lower electrodes. The actuator in one embodiment comprises a wire harness that includes insulated individual electrical wires or leads contained within a single sheath. The wire harness can exit the handle with the button and forms part of the cabled connection 5. The wires within the harness can provide electrical communication between the instrument and the electrosurgical generator and/or accessories thereof. In one aspect, once activated, the fuse button completes a circuit by electrically coupling at least two leads together. As such, an electrical path is then established from an electrosurgical generator to the actuator to supply RF energy to the instrument.

In some embodiments, electrode geometry on the conductive pads of the jaw assembly ensures that the sealing area completely encloses the distal portion of the cutting path. In accordance with various embodiments, the dimensions of the jaw surfaces are such that it is appropriately proportioned with regards to the optimal pressure applied to the tissue between the jaws for the potential force the force mechanism can create. Its surface area is also electrically significant with regards to the surface area contacting the tissue. This proportion of the surface area and the thickness of the tissue have been optimized with respect to its relationship to the electrical relative properties of the tissue. As such, in various embodiments, the conductive pads are flat and planar and are operationally arranged to transmit RF energy between the pads and through tissue between the pads with the RF energy supplied by an electrosurgical generator to optimize fusing of tissue therebetween. Also, in various embodiments, the total surface area of the conductive pad of the upper jaw is larger than the total surface area of the conductive pad of the lower jaw to optimize fusing and dissecting of tissue therebetween.

In accordance with various embodiments, an electrosurgical system can include an electrosurgical generator and an electrosurgical tool. The electrosurgical tool is used in open procedures where the ligation and division of vessels and tissue bundles are desired. The electrosurgical tool fuses vessels by delivering radio frequency (RF) energy to tissue captured between the jaws of the device and subsequently or simultaneously cuts the sealed tissue with the use of a user-actuated blade. The generator can provide an electrosurgery endpoint by determining the phase end point of a tissue to be treated. The electrosurgical system can include more than one electrosurgical tool for different electrosurgical operations and can include a variety of user interface features and audio/visual performance indicators. The electrosurgical system can also power conventional bipolar electrosurgical tools and direct current surgical appliances.

Turning now to some of the operational aspects of the electrosurgical tool or instrument described herein in accordance with various embodiments, once a vessel or tissue bundle has been identified for fusing, the first and second jaws are placed around the tissue. The handles are squeezed together and thereby pivot the first jaw towards the second jaw effectively clamping the tissue. The actuator 201 has a first or initial position in which the jaws 202 are in an open position and in one embodiment the first and second jaws opening defines about a 30 degree angle.

The force applied to the tissue by the jaws is translated through the support arm on one of the actuation handles. Once the preloaded force has been overcome, the support arm will begin to move closer to the opposite handle. When the engaged fuse position is reached and a small, e.g., minimum, amount of tissue is between the jaws the support spring ensures that the force applied to the electrodes of the jaws is near the lower end of the force range required for optimal vessel sealing. When a large, e.g., maximum, amount of tissue is placed in the jaws, the rocker arm spring ensures that the maximum amount of force applied does not exceed the maximum end of the force range used for optimal vessel sealing.

As such, the force and over compression regulation mechanism provides a minimum force, optimal for sealing vessels and tissue, that is maintained regardless of the amount of substance contained between the upper and lower jaws. This mechanism also reduces the risk that an extremely large amount of force is applied to the tissue. If too much force is applied to a vessel or tissue bundle, potential damage could occur. Thus, if a very small vessel or thin tissue bundle is clamped within the jaw, the instrument applies the minimum amount of force required to obtain a good tissue weld. The same is true with a very large vessel or tissue bundle. Since the travel of the jaw can vary greatly depending on tissue thickness, the force applied by the jaw is adjustable. The instrument is self-adjusting and automatic (no action from the user). The force and over compression regulation mechanism provides the self-adjustment, applying a specific range of force along the length of the electrode.

The continued manipulation of the handles pivots the handles to a location where the movable handle causes the depression of the fuse button. The depression of the fuse button causes the application of the radio frequency energy to the tissue between the jaws. Once the tissue has been fused and/or cut, the actuator is reopened by moving the handles apart. To cut tissue between the jaws, the user can actuate the blade trigger 21. When the blade trigger is moved proximally, the blade lever pivots, forcing the cutting blade distally. The cutting blade thus advances forward and divides the tissue. When the surgeon releases the blade trigger, the blade spring resets the cutting blade to its original position.

In accordance with various embodiments, the actuator 201 has a cutting or fuse position in which the jaws 202 are in a closed position and the blade trigger has been depressed advancing the cutting blade to its distal most position. In various embodiments, the blade trigger may be activated to cut tissue between the jaws and/or the fuse button or switch may be activated to fuse tissue between the jaws.

As described, in accordance with various embodiments, the instrument 3 has a first state in which the jaws 201 are spaced from each other and thus the handles 202 are also spaced from each other. The instrument is thus positioned to grasp tissue between the jaws. In the second state of the instrument, the jaws are proximate to each other to grasp tissue between the jaws and likewise the handles are proximate to each other. No RF energy is applied to the tissue. The surgeon can revert back to the first state by opening the jaws and thus positioning the jaws again to grasp the tissue or other tissue. In the third state of the instrument, the handles are moved further and closer to each other. However, the jaws remain in the same position as in the second state and thus over-compression of the tissue is avoided. Movement to the third state is needed to activate the switch or button to thereby apply RF energy to the tissue grasped between the jaws. Also, movement to the third state, releases the blade lock hook and thereby tissue grasped between the jaws can be cut through the activation of the blade lever. Movement to the third state also reduces the potential of unintentionally releasing the tissue. Also, inadvertent cutting of tissue or along the wrong tissue lines are avoided. Additionally, this state allows the application of constant and continuous predefined compression or range of compression on the tissue between the jaws before, during and after the activation of the RF energy, thereby enhancing the sealing or fusion of the tissue between the jaws.

In various embodiments, the lower handle pivoted and spaced away from the upper handle in an open position or first state defines an open distance between the lower handle and the upper handle and the lower handle proximate the upper handle in a closed position or second state defines a closed distance between the lower handle and the upper handle, the closed distance (D) being smaller than the open distance. In various embodiments, the lower handle proximate the upper handle in the fuse position defines a fuse distance between the lower handle and the upper handle, the fuse distance being smaller than the closed distance. In accordance with various embodiments, the fuse distance corresponds to or is greater than the height of the uncompressed support spring or the distance between the proximal end of the support arm and the lower handle housing.

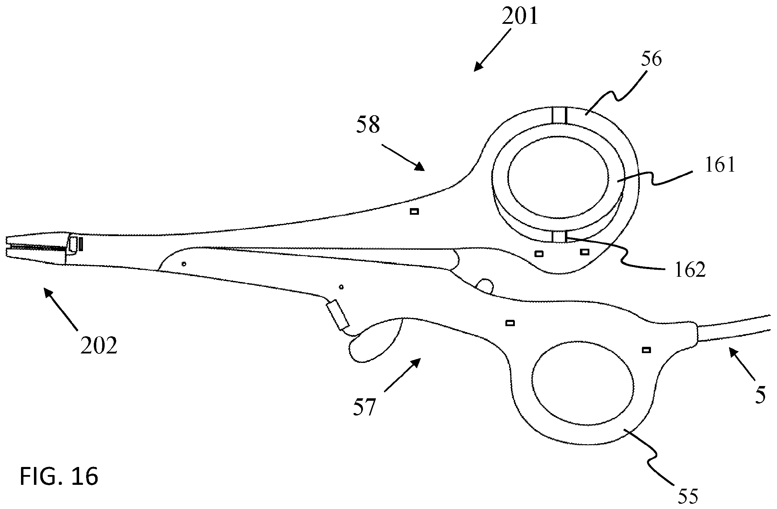

In reference to FIG. 16, in one embodiment, the upper handle 58 connected to the lower jaw 20 includes an upper ring handle or finger loop 56 and the lower handle 57 connected to the upper jaw 10 includes a lower ring handle or finger loop 55. In one embodiment the upper ring handle includes an internal loop 161 biased by a spring 162. The internal loop is spring loaded to the upper ring handle and moves vertically or perpendicular to the longitudinal axis. The spring applies a specific force to the upper handle to control the clamping force at the jaws. As such, over compression of the tissue and thus unintended tissue trauma is prevented. In one embodiment, the spring comes into play only when the jaws are closed. It is appreciated however that the internal loop can operationally not engaged by the surgeon for example grasping the outer portion of the ring handle or handle. As such, the internal loop would not be engaged and thus the clamping force would not be regulated by the spring connected to the loop. In various embodiments, a fuse button is disposed between the internal loop and the ring handle and thus in order to fuse tissue grasped between the jaws, the internal loop is engaged thereby engaging the spring. Various other components and/or combinations thereof are also applicable to the illustrated embodiment in FIG. 16 and vice versa.

In accordance with various embodiments, the gripping force generated between the jaws can vary along the length of the jaws from a relative maximum near the proximal end to a relative minimum near the distal end. The electrosurgical instrument is configured such that the forces are optimized along the length of the electrode portions of the jaws such that a predetermined force range for vessel sealing is maintained. A predetermined maximum amount of force utilized to obtain a proper vessel seal is not exceeded at the proximal end of the active electrodes (closest to the pivot). In addition, a gripping force at the distal most ends of the electrodes is greater than a predetermined minimum amount of force for optimal vessel sealing. The gripping force generated at every point along the jaws is within the range defined by the predetermined maximum force and the predetermined minimum force to achieve optimal sealing.

It should also be appreciated that springs are described throughout the description for ease of description. However, other biasing mechanisms could be used such as elastic bands, hydraulics, etc. It should be appreciated that the force and over compression mechanism avoids spring configurations such as springs or biasing mechanisms toward or near the proximal end of the handles and exposed or biased between both handles that causes a back force requiring a surgeon to overcome to close the handles and thus the jaws. Thus, with a surgeon performing multiple seals, such as twenty to fifty seals, in a single surgical procedure, hand fatigue can set in as the biasing mechanism would be sufficiently strong to ensure compression of the tissue at the jaws and also prevent a surgeon from over compressing the tissue. Additionally, a predetermined force or range a forces would also be difficult to control and predict given the different operational force and varying force that can be applied by a surgeon and various surgeons for different surgical procedures over varying time frames. Furthermore, interference with a surgeon's hand or operation may also occur if the bias mechanism is exposed near the proximal end of the instrument. Springs or bias mechanisms disposed near or within the jaws of the instrument can cause interference with tissue disposed or attempted to be grasped by the jaws and cause misalignment or non-parallel jaw closures which thereby may cause improper tissue sealing or cutting. Additionally, eschar or other types of buildup on the jaws may interfere with such bias mechanisms. Furthermore, placement of such biasing mechanisms may cause an increase in jaw size and thus potentially obstruct a surgeon's view of the fusion site or potentially interfere with the blade mechanism.

In one aspect, the determination of the end-point of the fusion process is given by monitoring the phase shift of voltage and current during the fusion process. In accordance with various embodiments, the application of RF energy via an electrosurgical generator in conjunction with the measuring or monitoring of phase shift are provided to fuse vessels and tissue in accordance with various embodiments of electrosurgical system. As such, the instrument generating the seal, fusion or connection of the tissue provides atraumatic contact to the connecting tissue and provides enough burst pressure, tensile strength, or breaking strength within the tissue.

In one embodiment, the generator initially determines the initial instrument impedance and/or capacitance (e.g., during plug-in of the instrument connector to the electrosurgical generator), where tolerances/changes in the instrument characteristics are then accounted for in the tissue measurement and endpoint determination process. This can allow for tissue measurement values which are independent of the ohmic and capacitive values and/or tolerances of a specific electrosurgical instrument.

Exemplary RF energy control process for the electrosurgical generator and associated electrosurgical instrument for fusing or sealing tissue in accordance with various embodiments is provided in which RF energy is supplied by the generator through the connected electrosurgical instrument or tool. The generator monitors at least the phase and/or change of phase of the supplied RF energy. In various embodiments, if a phase crossing or polarity change from positive to negative or negative to positive is encountered, a phase stop is determined. The phase stop in various embodiments includes a predefined phase angle and/or change of phase angle that indicates an optimal tissue seal or fusion endpoint and/or is based on a determined tissue property such as size, permittivity, conductivity and/or applied voltage, current and/or power. The generator continues to monitor at least the phase and/or change of phase of the supplied RF energy. If the phase stop is reached or exceeded, the process is done or termination procedures are initiated and/or RF energy supplied by the generator is stopped.

In accordance with various embodiments, at a detected phase crossing or polarity change, the generator identifies the voltage level of the supplied RF energy and, depending on the determined voltage level a specific course of action is selected. For example, if the identified voltage is less than 50 volts, the voltage level is set to a constant value of 25 volts and the RF energy continues to be supplied until the monitored phase angle reaches a phase angle end point value of -7 degrees. If the identified voltage is greater than or equal to 50 volts but less than or equal to 60 volts, the voltage level is held constant at the identified voltage and RF energy continues to be supplied until the monitored phase angle reaches a phase angle end point value of -14 degrees. If the identified voltage is greater than 60 volts, the voltage level is held constant at the identified voltage and RF energy continues to be supplied until the sensed phase angle reaches a phase angle end point value of -14.5 degrees.

In certain embodiments, an electrosurgical fusion/sealer and divider tool comprises a handle assembly, a jaw assembly, and a force and over compression regulation mechanism. The handle assembly comprises two pivotably movable handles. The jaw assembly comprises a first jaw and a second jaw. The first jaw has an inner surface, an outer surface, and at least one electrode disposed on the inner surface. The second jaw has an inner surface, an outer surface, and at least one electrode disposed on the inner surface. The jaw assembly is actuatable by movement of the handle assembly from an open configuration in which the inner surface of the first jaw is spaced apart from the inner surface of the second jaw to a closed configuration in which the inner surface of the first jaw is proximate the inner surface of the second jaw. The force and over compression regulation mechanism is configured such that in the closed configuration, the jaw assembly delivers a gripping force between the first jaw and the second jaw between a predetermined minimum force and a predetermined maximum force.

In other embodiments, the jaw assembly comprises a blade. The blade is longitudinally advanceable along the inner surface of the first jaw along a cutting path defined between a retracted position adjacent the proximal end and an advanced position between the proximal end and the distal end. The blade is advanceable by movement of a blade trigger on the handle assembly. The at least one electrode on the first jaw and the at least one electrode on the second jaw have opposing polarity and define a fusion and/or dissection area enclosing the cutting or dividing path. In various embodiments, the jaw assembly is provided to fuse or seal when RF energy is applied and subsequently to cut tissue between the jaw members using a mechanical cutting blade.

In some embodiments, the electrosurgical tool can be used in a system which monitors various operational parameters and determines a radiofrequency endpoint based on phase angle and/or change of phase angle. The electrosurgical tool fuses vessels by delivering radio frequency (RF) energy to tissue grasped between the jaws of the device.

Further examples of the electrosurgical generator, unit, instruments and connections there between and operations and/or functionalities thereof are described in US Patent Application Nos. 12/416,668, 12/416,751, 12/416,695, 12/416,765, and 12/416,128. Certain aspects of these electrosurgical generators, tools and systems are discussed herein, and additional details and examples with respect to various embodiments are described in US Provisional Application Nos. 61/994,215, 61/944,185, 61/944,192, 61/994,415, 62/005,009, and US Patent Application No. 14/848,116.

The above description is provided to enable any person skilled in the art to make and use the surgical tools and perform the methods described herein and sets forth the best modes contemplated by the inventors of carrying out their inventions. Various modifications, however, will remain apparent to those skilled in the art. Different embodiments or aspects of such embodiments may be shown in various figures and described throughout the specification. However, it should be noted that although shown or described separately each embodiment and aspects thereof may be combined with one or more of the other embodiments and aspects thereof unless expressly stated otherwise. It is merely for easing readability of the specification that each combination is not expressly set forth.

Although the present invention has been described in certain specific aspects, many additional modifications and variations would be apparent to those skilled in the art. It is therefore to be understood that the present invention may be practiced otherwise than specifically described, including various changes in the size, shape and materials, without departing from the scope of the present invention as it is defined by the following claims.

## Claims

1. An electrosurgical instrument (201) comprising:
a lower handle (57) having a lower handle housing;
an upper jaw (10) comprising an upper jaw support (12) and an upper conductive pad (14), the upper jaw support having a circular opening and being coupled to the lower handle (57);
an upper handle (58) having a housing with a cavity disposed within the housing;
a lower jaw (11) comprising a lower jaw support (17) and a lower conductive pad (15), the lower conductive pad and the upper conductive pad being operationally arranged to transmit RF energy between the upper conductive pad and the lower conductive pad, the lower jaw support having a non-circular opening (19) and being coupled to the upper handle (58), and the upper and lower handles (57, 58) being arranged to be brought together from an open position, where the upper and lower jaws (10, 11) are open, to a closed position where the upper and lower jaws (10, 11) are closed; and
a central pivot (50) having a circular end, a non-circular end and an opening, the circular end of the central pivot inserted and connected to the circular opening in the upper jaw support (12) and the non-circular end inserted and connected to the non-circular opening in the lower jaw support (17), the circular end is configured to allow the upper jaw support (12) to pivot about the central pivot (50) and the non-circular end is configured to resist pivoting of the lower jaw support (17) about the central pivot (50) such that as the upper jaw (10) connected to the upper jaw support (12) is pivotable from the open position to the closed position and vice versa, the lower jaw (11) remains stationary due to its connection to the lower jaw support (17) and the non-circular end of the central pivot (50);
wherein the upper handle (58) comprises:
a blade trigger (21) connected to the upper handle (58);
a blade slider (24), in which movement of the blade trigger (21) moves the blade slider (24), the blade slider (24) being movable longitudinally through the opening of the central pivot and a channel in the upper and lower jaws (10,11) extending longitudinally along the upper and lower jaws (10,11) with the lower jaw support further comprising a guide channel dimensioned to receive and support the blade slider, the guide channel aligning and supporting the longitudinal movement of the blade slider (24).

2. The instrument (201) of claim 1 wherein the lower jaw support (17) further comprises a proximal pin channel at a proximal end of the lower jaw support (17), the proximal pin channel dimensioned to receive and support a pin connecting the blade slider (24) to a blade lever arm (23), the proximal pin channel aligning and supporting the longitudinal movement of the blade slider (24).

3. The instrument (201) of claim 2 further comprises a blade spring (26) connected to the blade trigger (21), the blade spring (26) biasing the blade trigger proximally in which the blade trigger (21) moving proximally moves the blade lock hook (25) to reengage the blade lock hook (25) with the blade trigger (21).

4. The instrument (201) of claim 3 wherein the lower handle (57) comprises a jaw support joint (40) disposed between the upper jaw (10) and the lower handle housing, the j aw support joint (40) having a cavity and a distal portion of a support arm (42) is connected to jaw support joint (40) and is enclosed within the cavity of the jaw support joint (40) and a support spring (41) connected to the support arm (42) to apply a force on the upper and lower jaws (10, 11).

5. The instrument (201) of claim 4 further comprising a switch (33) arranged to activate through contact with the lower handle housing of the lower handle (57) to connect to a supply of RF energy.

6. The instrument (201) of claim 5 wherein the jaw support joint (40) is aligned with the upper jaw (10) and the lower handle (57) housing when the upper handle (58) is spaced from the lower handle (57) and the lower handle housing pivots relative to the jaw support joint (40) when the upper and lower handles (57, 58) are proximate each other in a fuse position.

7. The instrument (201) of claim 6 wherein the fuse position is a position in which the upper and lower handles (57, 58) are closer to each other than in the closed position and the switch is activated.

8. The instrument (201) of claim 7 wherein the lower handle housing has a curved surface that mates with a curved surface of the jaw support joint (40) with the curved surface of the lower handle housing being a mirror image of the curved surface of the jaw support joint (40) to externally support the decoupling of the lower handle housing from the jaw support joint (40).

9. The instrument (201) of claim 1 or 3 wherein the lower jaw (11) comprises a conductive post.

10. The instrument (201) of claim 9 wherein the conductive post is positioned adjacent to the channel in the lower jaw (11) and configured to strengthen the channel.

11. The instrument (201) of claim 10 further comprising a lower assembly spacer (16) positioned between the lower jaw support (17) and the lower conductive pad (15).

12. The instrument (201) of claim 11 further comprising an upper assembly spacer (13) positioned between the upper jaw support (12) and the upper conductive pad (14).

13. The instrument (201) of any one of claims 1-12 wherein the upper conductive pad (14) and the lower conductive pad (15) are flat and planar.

14. The instrument (201) of claim 1 or 3 wherein the upper handle (58) comprises an upper ring (56) at a proximal end of the upper handle and an inner loop (161) disposed and movable within the upper ring.

15. The instrument (201) of claim 14 wherein the upper handle comprises a spring (162) disposed between the inner loop (161) and the upper ring (56) and biasing the inner loop (171) relative to the upper ring (56) and in a direction transverse to a longitudinal axis of the electrosurgical instrument.

## Patentansprüche

1. Elektrochirurgisches Instrument (201), das Folgendes umfasst:
einen unteren Griff (57), der ein unteres Griffgehäuse aufweist;
eine obere Backe (10), die einen Träger der oberen Backe (12) und eine obere leitfähige Auflage (14) umfasst, wobei der Träger der oberen Backe eine kreisförmige Öffnung aufweist und mit dem unteren Griff (57) verbunden ist;
einen oberen Griff (58), der ein Gehäuse mit einem Hohlraum aufweist, der innerhalb des Gehäuses angeordnet ist;
eine untere Backe (11), die einen Träger der unteren Backe (17) und eine untere leitfähige Auflage (15) umfasst, wobei die untere leitfähige Auflage und die obere leitfähige Auflage funktionsfähig angeordnet sind, um HF-Energie zwischen der oberen leitfähigen Auflage und der unteren leitfähigen Auflage zu übertragen, wobei der Träger der unteren Backe eine nicht-kreisförmige Öffnung (19) aufweist und mit dem oberen Griff (58) verbunden ist und der obere und untere Griff (57, 58) angeordnet sind, um von einer offenen Position, wo die obere und untere Backe (10, 11) offen sind, zu einer geschlossenen Position, wo die obere und untere Backe (10, 11) geschlossen sind, zusammengebracht zu werden; und
einen zentralen Drehzapfen (50), der ein kreisförmiges Ende, ein nicht-kreisförmiges Ende und eine Öffnung aufweist, wobei das kreisförmige Ende des zentralen Drehzapfens in die kreisförmige Öffnung in dem Träger der oberen Backe (12) eingeführt ist und damit verbunden ist und das nicht-kreisförmiges Ende in die nicht-kreisförmige Öffnung in dem Träger der unteren Backe (17) eingeführt ist und damit verbunden ist, wobei das kreisförmige Ende konfiguriert ist, um ein Schwenken des Trägers der oberen Backe (12) um den zentralen Drehzapfen (50) herum zu ermöglichen, und das nicht-kreisförmige Ende konfiguriert ist, um einem Schwenken des Trägers der unteren Backe (17) um den zentralen Drehzapfen (50) herum entgegenzuwirken, sodass die obere Backe (10), die mit dem Träger der oberen Backe (12) verbunden ist, von der offenen Position zu der geschlossenen Position und umgekehrt schwenkbar ist, die untere Backe (11) aufgrund ihrer Verbindung mit dem Träger der unteren Backe (17) und dem nicht-kreisförmigen Ende des zentralen Drehzapfens (50) unbeweglich bleibt;
wobei der obere Griff (58) Folgendes umfasst:
einen Klingenauslöser (21), der mit dem oberen Griff (58) verbunden ist;
einen Klingenschieber (24), wobei eine Bewegung des Klingenauslösers (21) den Klingenschieber (24) bewegt, wobei der Klingenschieber (24) längs durch die Öffnung des zentralen Drehzapfens und einen Kanal in der oberen und unteren Backe (10, 11), der sich längs entlang der oberen und unteren Backe (10, 11) erstreckt, verschiebbar ist, wobei der Träger der unteren Backe weiter einen Führungskanal umfasst, der zur Aufnahme und zum Tragen des Klingenschiebers dimensioniert ist, wobei der Führungskanal die Längsbewegung des Klingenschiebers (24) ausrichtet und unterstützt.

2. Instrument (201) nach Anspruch 1, wobei der Träger der unteren Backe (17) weiter einen proximalen Stiftkanal an einem proximalen Ende des Trägers der unteren Backe (17) umfasst, wobei der proximale Stiftkanal zur Aufnahme und zum Tragen eines Stifts dimensioniert ist, der den Klingenschieber (24) mit einem Klingenhebelarm (23) verbindet, wobei der proximale Stiftkanal die Längsbewegung des Klingenschiebers (24) ausrichtet und unterstützt.

3. Instrument (201) nach Anspruch 2, das weiter eine Klingenfeder (26) umfasst, die mit dem Klingenauslöser (21) verbunden ist, wobei die Klingenfeder (26) den Klingenauslöser proximal vorspannt, wobei der Klingenauslöser (21), der sich proximal bewegt, den Klingenverriegelungshaken (25) bewegt, damit der Klingenverriegelungshaken (25) erneut in den Klingenauslöser (21) eingreift.

4. Instrument (201) nach Anspruch 3, wobei der untere Griff (57) Folgendes umfasst: eine Backentragverbindung (40), die zwischen der oberen Backe (10) und dem unteren Griffgehäuse angeordnet ist, wobei die Backentragverbindung (40) einen Hohlraum aufweist, und ein distaler Teil des Tragarms (42) mit der Backentragverbindung (40) verbunden ist und innerhalb des Hohlraums der Backentragverbindung (40) eingeschlossen ist, und eine Stützfeder (41), die mit dem Tragarm (42) verbunden ist, um eine Kraft auf die obere und untere Backe (10, 11) auszuüben.

5. Instrument (201) nach Anspruch 4, das weiter einen Schalter (33) umfasst, der zur Aktivierung durch den Kontakt mit dem unteren Griffgehäuse des unteren Griffs (57) angeordnet ist, um mit einer Zufuhr von HF-Energie zu verbinden.

6. Instrument (201) nach Anspruch 5, wobei die Backentragverbindung (40) mit der oberen Backe (10) und dem unteren Griff (57)-Gehäuse ausgerichtet ist, wenn der obere Griff (58) von dem unteren Griff (57) beabstandet ist, und das untere Griffgehäuse in Bezug auf die Backentragverbindung (40) geschwenkt wird, wenn der obere und untere Griff (57, 58) nahe zueinander in einer Fusionsposition vorliegen.

7. Instrument (201) nach Anspruch 6, wobei die Fusionsposition eine Position ist, in der der obere und untere Griff (57, 58) näher zueinander vorliegen als in der geschlossenen Position und der Schalter aktiviert ist.

8. Instrument (201) nach Anspruch 7, wobei das untere Griffgehäuse eine gekrümmte Oberfläche aufweist, die mit einer gekrümmten Oberfläche der Backentragverbindung (40) zusammenpasst, wobei die gekrümmte Oberfläche des unteren Griffgehäuses ein Spiegelbild der gekrümmten Oberfläche der Backentragverbindung (40) ist, um die Loslösung des unteren Griffgehäuses von der Backentragverbindung (40) extern zu unterstützen.

9. Instrument (201) nach Anspruch 1 oder 3, wobei die untere Backe (11) einen leitfähigen Stab umfasst.

10. Instrument (201) nach Anspruch 9, wobei der leitfähige Stab benachbart zu dem Kanal in der unteren Backe (11) positioniert ist und konfiguriert ist, um den Kanal zu verstärken.

11. Instrument (201) nach Anspruch 10, das weiter einen unteren Anordnungsabstandshalter (16) umfasst, der zwischen dem Träger der unteren Backe (17) und der unteren leitfähigen Auflage (15) positioniert ist.

12. Instrument (201) nach Anspruch 11, das weiter einen oberen Anordnungsabstandshalter (13) umfasst, der zwischen dem Träger der oberen Backe (12) und der oberen leitfähigen Auflage (14) positioniert ist.

13. Instrument (201) nach einem der Ansprüche 1-12, wobei die obere leitfähige Auflage (14) und die untere leitfähige Auflage (15) flach und planar sind.

14. Instrument (201) nach Anspruch 1 oder 3, wobei der obere Griff (58) einen oberen Ring (56) an einem proximalen Ende des oberen Griffs und einen Innenring (161), der innerhalb des oberen Rings angeordnet und beweglich ist, umfasst.

15. Instrument (201) nach Anspruch 14, wobei der obere Griff eine Feder (162) umfasst, die zwischen dem Innenring (161) und dem oberen Ring (56) angeordnet ist und den Innenring (171) in Bezug auf den oberen Ring (56) und in einer Richtung quer zu einer Längsachse des elektrochirurgischen Instruments vorspannt.

## Revendications

1. Instrument électrochirurgical (201) comprenant :
une poignée inférieure (57) ayant un boîtier de poignée inférieure ;
une mâchoire supérieure (10) comprenant un support de mâchoire supérieure (12) et un patin conducteur (14), le support de mâchoire supérieure ayant une ouverture circulaire et étant couplé à la poignée inférieure (57) ;
une poignée supérieure (58) ayant un boîtier et une cavité placée dans le boîtier ; une mâchoire inférieure (11) comprenant un support de mâchoire inférieure (17) et un patin conducteur inférieur (15), le patin conducteur inférieur et le patin conducteur supérieur étant disposés de manière opérationnelle pour transmettre de l'énergie RF entre le patin conducteur supérieur et le patin conducteur inférieur, le support de mâchoire inférieure ayant une ouverture non circulaire (19) et étant couplé à la poignée supérieure (58), et les poignées supérieure et inférieure (57, 58) étant disposées pour être réunies d'une position ouverte, lorsque les mâchoires supérieure et inférieure (10, 11) sont ouvertes, à une position fermée lorsque les mâchoires supérieure et inférieure (10, 11) sont fermées ; et
un pivot central (50) ayant une extrémité circulaire, une extrémité non circulaire et une ouverture, l'extrémité circulaire du pivot central étant insérée et raccordée à l'ouverture circulaire dans le support de mâchoire supérieure (12) et l'extrémité non circulaire étant insérée et raccordée à l'ouverture non circulaire dans le support de mâchoire inférieure (17), l'extrémité circulaire est configurée pour permettre au support de la mâchoire supérieure (12) de pivoter autour du pivot central (50) et l'extrémité non circulaire est configurée pour résister au pivotement du support de mâchoire inférieure (17) autour du pivot central (50) de sorte qu'au fur et à mesure que la mâchoire supérieure (10) raccordée au support de mâchoire supérieure (12) peut pivoter de la position ouverte à la position fermée et vice versa, la mâchoire inférieure (11) demeure stationnaire du fait de son raccordement au support de mâchoire inférieure (17) et à l'extrémité non circulaire du pivot central (50) ;
dans lequel la poignée supérieure (58) comprend :
une gâchette de lame (21) raccordée à la poignée supérieure (58) ;
une glissière de lame (24), dans laquelle le mouvement de la gâchette de lame (21) déplace la glissière de lame (24), la glissière de lame (24) étant amovible longitudinalement à travers l'ouverture du pivot central et un canal dans les mâchoires supérieure et inférieure (10, 11) s'étendant longitudinalement le long des mâchoires supérieure et inférieure (10, 11) avec le support de mâchoire inférieure comprenant en outre un canal de guidage dimensionné pour recevoir et soutenir la glissière de lame, le canal de guidage alignant et soutenant le mouvement longitudinal de la glissière de lame (24).

2. Instrument (201) selon la revendication 1, dans lequel le support de mâchoire inférieure (17) comprend en outre un canal de goupille proximal à une extrémité proximale du support de mâchoire inférieure (17), le canal de goupille proximal étant dimensionné pour recevoir et soutenir une goupille raccordant la glissière de lame (24) à un bras de levier de lame (23), le canal de goupille proximal alignant et soutenant le mouvement longitudinal de la glissière de lame (24).

3. Instrument (201) selon la revendication 2, comprenant en outre un ressort de lame (26) raccordé à la gâchette de lame (21), le ressort de lame (26) sollicitant la gâchette de lame proximalement en vertu de quoi la gâchette de lame (21) se déplaçant proximalement déplace le crochet de verrouillage (25) afin de réengager le crochet de verrouillage de lame (25) avec la gâchette de lame (21).

4. Instrument (201) selon la revendication 3, dans lequel la poignée inférieure (57) comprend un joint de support de mâchoire (40) placé entre la mâchoire supérieure (10) et le boîtier de la poignée inférieure, le joint de support de mâchoire (40) ayant une cavité et une partie distale d'un bras de support (42) est raccordée au joint de support de mâchoire (40) et est enfermée dans la cavité du joint de support de mâchoire (40) et un ressort de support (41) raccordé au bras de support (42) pour appliquer une force sur les mâchoires supérieure et inférieure (10, 11).

5. Instrument (201) selon la revendication 4, comprenant en outre un interrupteur (33) disposé afin de s'activer par contact avec le boîtier de poignée inférieure de la poignée inférieure (57) pour se connecter à une alimentation en énergie RF.

6. Instrument (201) selon la revendication 5, dans lequel le joint de support de mâchoire (40) est alignée avec la mâchoire supérieure (10) et le boîtier de la poignée inférieure (57) lorsque la poignée supérieure (58) est espacée de la poignée inférieure (57) et que le boîtier de la poignée inférieure pivote par rapport au joint de support de mâchoire (40) lorsque les poignées supérieure et inférieure (57, 58) sont proches l'une de l'autre dans une position de fusion.

7. Instrument (201) selon la revendication 6, dans lequel la position de fusion est une position dans laquelle les poignées supérieure et inférieure (57, 58) sont plus proches l'une de l'autre que dans la position fermée et que l'interrupteur est activé.

8. Instrument (201) selon la revendication 7, dans lequel le boîtier de la poignée inférieure a une surface courbée qui s'apparie à une surface courbée du joint de support de mâchoire (40) avec la surface courbée du boîtier de la poignée inférieure étant une image miroir de la surface courbée du joint de support de mâchoire (40) afin de soutenir extérieurement le découplage du boîtier de la poignée inférieure du joint de support de mâchoire (40).

9. Instrument (201) selon la revendication 1 ou 3, dans lequel la mâchoire inférieure (11) comprend une tige conductrice.

10. Instrument (201) selon la revendication 9, dans lequel la tige conductrice est positionnée adjacente au canal dans la mâchoire inférieure (11) et configurée pour renforcer le canal.

11. Instrument (201) selon la revendication 10, comprenant en outre une pièce d'écartement d'ensemble inférieur (16) positionnée entre le support de la mâchoire inférieure (17) et le patin conducteur inférieur (15).

12. Instrument (201) selon la revendication 11, comprenant en outre une pièce d'écartement d'ensemble supérieur (13) positionnée entre le support de la mâchoire supérieure (12) et le patin conducteur supérieur (14).

13. Instrument (201) selon l'une quelconque des revendications 1-12, dans lequel le patin conducteur supérieur (14) et le patin conducteur inférieur (15) sont plats et planaires.

14. Instrument (201) selon la revendication 1 ou 3, dans lequel la poignée supérieure (58) comprend une bague supérieure (56) à une extrémité proximale de la poignée supérieure et une boucle interne (161) placée et amovible dans la bague supérieure.

15. Instrument (201) selon la revendication 14, dans lequel la poignée supérieure comprend un ressort (162) placé entre la boucle interne (161) et la bague supérieure (56) et sollicitant la boucle interne (171) par rapport à la bague supérieure (56) et dans une direction transversale à un axe longitudinal de l'instrument électrochirurgical.
